# FASCICULE DE BREVET EUROPEEN

(11) **EP 0 712 617 B1**
(45) Date de publication et mention de la délivrance du brevet: **29.09.1999**
(21) Numéro de dépôt: 95420323.8
(22) Date de dépôt: 17.11.1995
(51) Int. Cl.: A61F 2/40

(54) **Prothèse humérale à sphère**
Sphärische Oberarmprothese
Spherical humeral prosthesis

(30) Priorité: 18.11.1994 FR 9414052
(43) Date de publication de la demande: 22.05.1996
(73) Titulaire: TORNIER SA, 38330 Saint-Ismier (FR)
(72) Inventeur: Walch, Gilles, F-69003 Lyon (FR); Boileau, Pascal, F-06300 Nice (FR)
(74) Mandataire: Myon, Gérard Jean-Pierre

(56) Documents cités:
- CH-A- 507 704
- FR-A- 2 685 633
- FR-A- 2 689 756
- FR-A- 2 697 996
- US-A- 4 106 128

## Description

La présente invention est relative à des perfectionnements à apporter aux prothèses de l'extrémité supérieure de l'humérus, du genre comprenant une queue qui s'ancre dans le canal huméral et une calotte hémisphérique propre à coopérer avec la glène de l'épaule.

La prothèse suivant l'invention associée à une glène prothétique permet le traitement chirurgical notamment des affections dégénératives de l'arthrose gléno-humérale, mais encore celui d'autres affections. De manière isolée, la prothèse humérale suivant l'invention est destinée à des indications larges telles que les fractures céphalo-tubérositaires non accessibles à une chirurgie conservatrice, mais aussi à tout syndrome douloureux de l'épaule lié à une destruction isolée de la tête humérale.

Bien entendu, on connaît déjà des prothèses destinées à de tels traitements, telles que celles dites de NEER. Toutefois, une telle prothèse est monobloc, de telle sorte qu'il faut posséder en stock un grand nombre d'implants pour répondre aux exigences anatomiques des divers patients. De plus, ces prothèses présentent une seule taille de tige et de calotte hémisphérique, cette dernière ne pouvant pas être déportée comme cela est nécessaire dans certains cas. De plus, cette calotte présente une inclinaison fixe et elle ne couvre pas de manière satisfaisante le plan de coupe de l'extrémité de l'humérus, de telle sorte qu'il faut adapter l'os à la prothèse avec toutes les conséquences anatomiques que cela entraîne.

On connaît aussi, d'après le document SUISSE 507 704, une queue de prothèse de hanche qui comprend à l'une de ses extrémités une collerette pourvue d'une surface d'appui destinée à faire reposer ladite collerette sur le restant de la partie osseuse.

Un pivot est issu de la collerette en déterminant un angle d'inclinaison par rapport à la surface d'appui. Le pivot est de préférence conique et présente sur sa périphérie des moyens de retenue tels que des nervures, des dents, des saillies ou analogues.

Sur cette queue de prothèse, on peut venir fixer différentes rotules qui sont toutes solidaires d'un col prévu d'une inclinaison variable. La fixation est réalisée au moyen du pivot et des moyens de retenue.

L'utilisation de plusieurs rotules permet de définir le plus favorablement l'angle d'inclinaison, l'angle d'antéversion, et le déport latéral de la calotte sphérique. Une telle prothèse comporte certains inconvénients en ce qui concerne la collerette qui empêche à la prothèse un maintien optimum dans la métaphyse et réduisant nettement le dépassement spatial de la calotte sphérique.

On connaît également d'après la demande de brevet français N° 2 685 633 appartenant au demandeur une prothèse humérale modulaire, comportant des éléments dont une queue humérale d'ancrage pourvue d'une partie métaphysaire formée d'une face d'appui oblique. Une entretoise en forme de coin dont l'une des faces extrêmes s'ajuste sur la face d'appui de la queue. Une calotte hémisphérique humérale dont la base se fixe par rapport à la deuxième face extrême de l'entretoise. Cette fixation, qui est excentrée par rapport à l'axe géométrique de la calotte, permet un réglage angulaire de cette dernière par rapport à l'entretoise. La calotte et la queue sont verrouillées à l'entretoise à la position désirée.

Une telle prothèse comporte certains inconvénients en ce qui concerne, la hauteur de placement de la calotte, le réglage indexé mais non continu de l'inclinaison de la calotte, le déport dans le plan de coupe osseuse, et le réglage de la rétro-torsion de la calotte indépendemment de la position de la queue.

Les perfectionnements qui font l'objet de la présente invention visent à permettre la réalisation d'une prothèse humérale qui remédie aux inconvénients ci-dessus.

La prothèse humérale suivant la présente invention comprend une queue humérale qui est pourvue dans sa partie métaphysaire d'un logement à fond sphérique dans lequel est introduite une sphère prévue pour recevoir une calotte hémisphérique, tandis que des moyens de serrage permettent leur immobilisation dans une position angulaire déterminée par rapport à l'axe de la queue humérale.

Le dessin annexé, donné à titre d'exemple, permettra de mieux comprendre l'invention, les caractéristiques qu'elle présente et les avantages qu'elle est susceptible de procurer :
Fig. 1 est une vue en perspective éclatée illustrant la prothèse humérale suivant la présente invention.
Fig. 2 est une coupe montrant en détail la mise en place et la retenue de la calotte hémisphérique à l'intérieur de la queue de la prothèse humérale.
Fig. 3 est une coupe suivant III-III (fig. 2) représentant les moyens de serrage pour l'immobilisation de la sphère et de la calotte par rapport à l'axe de la prothèse humérale.
Fig. 4 et 5 sont des vues illustrant une première variante de la prothèse humérale suivant l'invention.
Fig. 6 et 7 sont des vues représentant une seconde variante de la prothèse humérale suivant la présente invention.
Fig. 8 à 13 sont des vues montrant une troisième variante de la prothèse humérale suivant la présente invention.

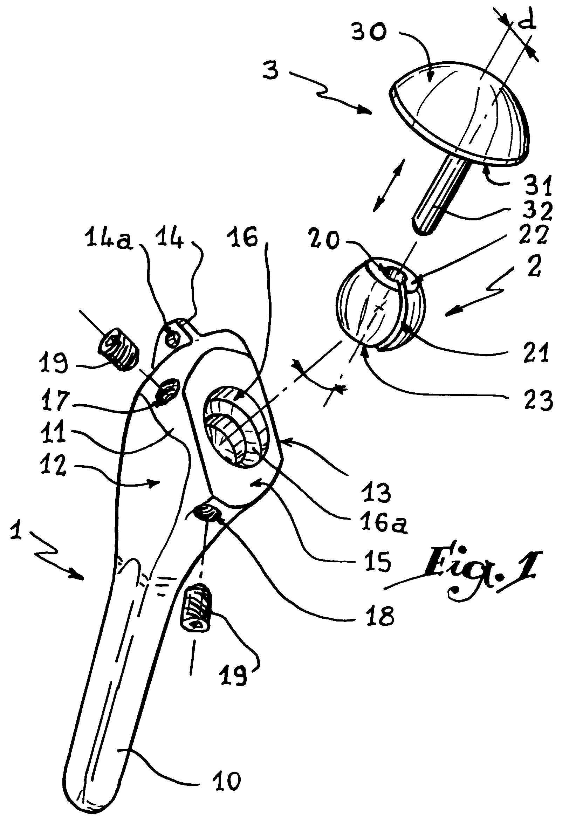

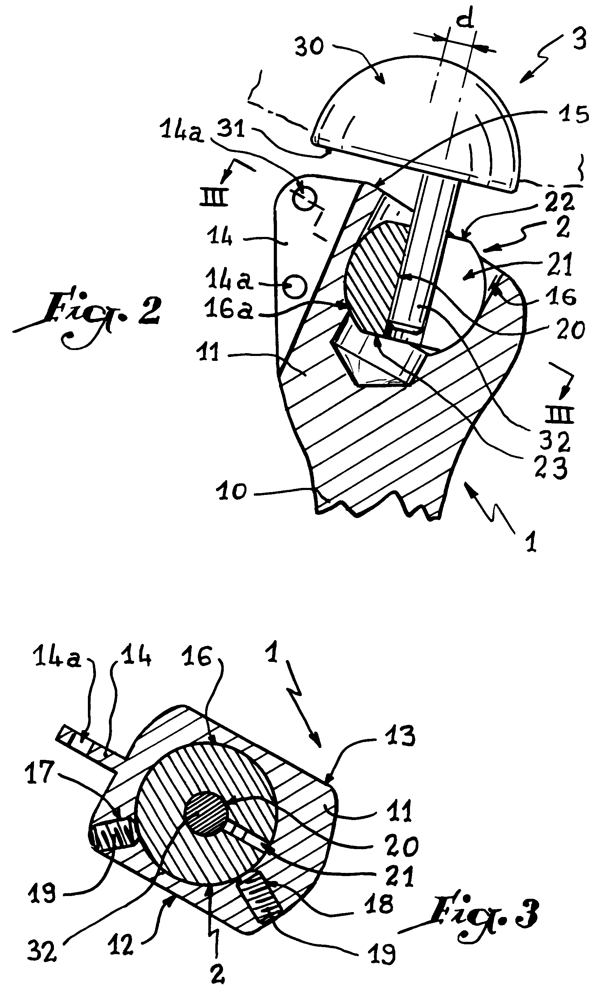

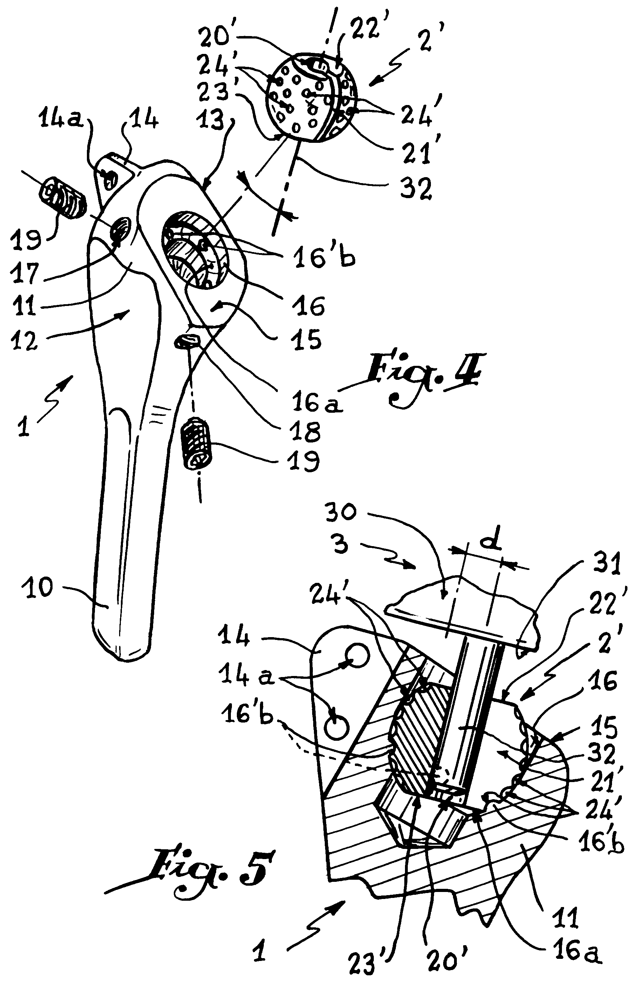

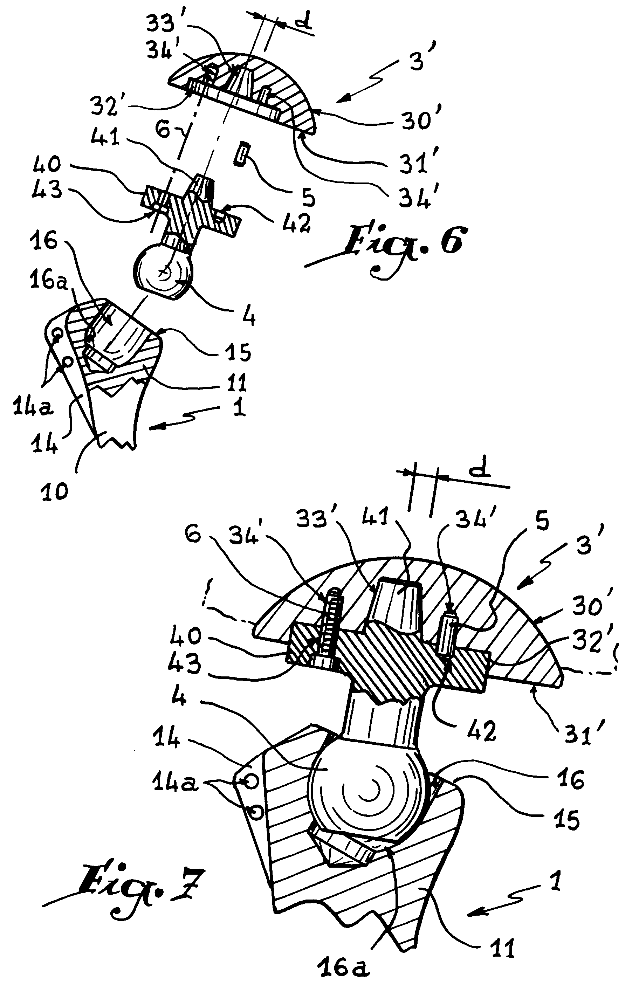

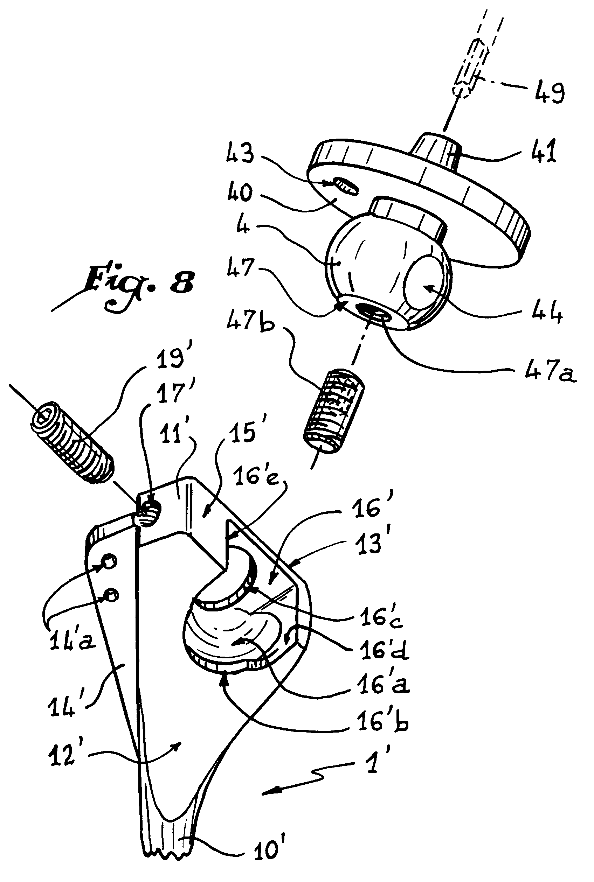

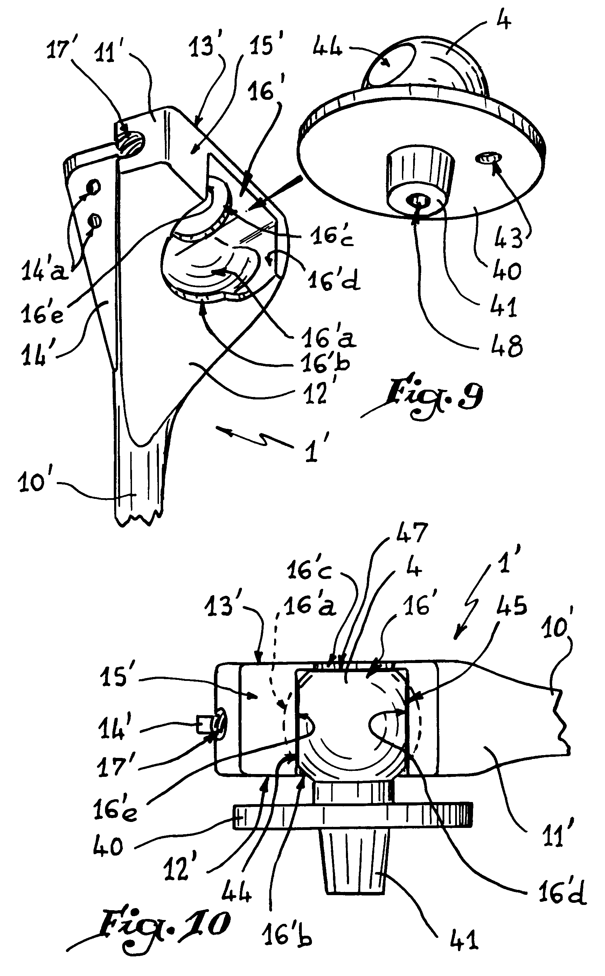

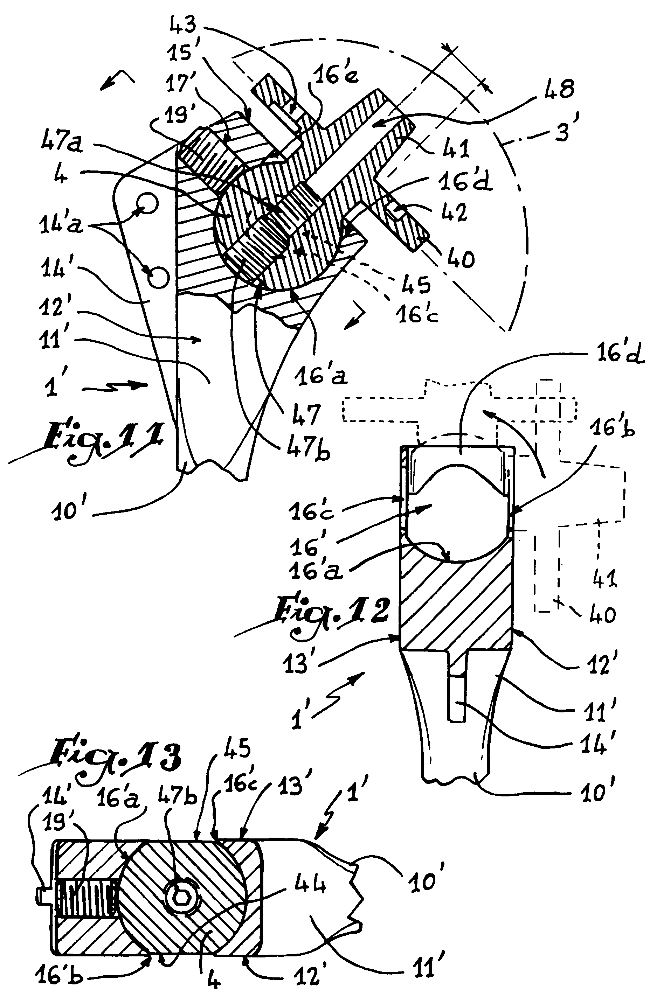

On a représenté en fig. 1 et 2 les trois éléments de la prothèse modulaire suivant l'invention, à savoir une queue 1, une sphère 2 et une calotte hémisphérique 3.

La queue 1 comprend une tige 10 de section circulaire qui s'engage dans le canal huméral et une partie métaphysaire 11 présentant un profil évasé. Dans le plan sagittal, la partie métaphysaire 11 comporte des flans 12 et 13 légèrement inclinés. En outre, la partie métaphysaire 11 est pourvue d'un ou plusieurs ailerons 14 disposé sur la face externe et/ou latérale de la tige 10 et qui comprend au moins une perforation 14a permettant, dans le cas de fracture, une reconstitution de l'extrémité supérieure de l'humérus autour de la prothèse.

La tige 10, et plus particulièrement la partie métaphysaire 11, se termine par une face inclinée 15 par rapport à l'axe longitudinal de ladite tige. La face inclinée 15 est percée d'un logement borgne 16 à profil cylindrique. Le fond du logement cylindrique 16 présente une portée sphérique 16a destinée à servir d'appui à la sphère 2, comme on le verra mieux plus loin.

La partie métaphysaire 11 est percée de deux trous taraudés 17, 18 qui débouchent à l'intérieur du logement cylindrique 16 et qui sont prévus pour recevoir chacun une vis de pression 19 sans tête. Les axes des trous 17 et 18 sont prévus obliques et inclinés par rapport aux axes du logement 16.

La sphère 2 est percée en son milieu d'un alésage 20 dans lequel débouche une fente radiale 21 donnant une certaine élasticité à ladite sphère. Les pôles de la sphère 2 comportent respectivement un plat 22, 23 diminuant son encombrement à l'intérieur du logement 16 de la partie métaphysaire 11.

La calotte hémisphérique 3 comprend une face extérieure 30 parfaitement polie, limitée par une base 31. Cette dernière comprend perpendiculairement un axe cylindrique 32 qui est décalé latéralement d'une distance d par rapport à son milieu permettant d'offrir la possibilité d'un excentrage de la calotte 3. De plus l'axe 32 peut présenter un profil extérieur conique dont le diamètre le moins large se trouve à l'opposé de la base 31.

Une fois la sphère 2 introduite dans le logement 16 en appui contre la portée sphérique 16a, on comprend aisément qu'il suffise de mettre en place la calotte hémisphérique 3 de manière que l'axe 32 coopère avec l'alésage 20 de ladite sphère. La hauteur de l'axe 32 est suffisante pour pouvoir régler la distance séparant la calotte 3 par rapport à la tige 1 de la prothèse humérale. En outre, la sphère 2 permet un réglage angulaire de la calotte 3 dans toutes les directions possibles par rapport à l'axe de la queue 1. Enfin, le décalage latéral de l'axe 32 par rapport au milieu de la base 31 de la calotte 3 permet un dernier réglage autour dudit axe afin de l'excentrer pour qu'elle puisse coopérer avec la glène de l'épaule.

Dès que tous les réglages angulaires sont réalisés, le chirurgien serre les vis 19 à l'intérieur des trous 17 et 18 de manière à ce qu'elles viennent en pression contre la sphère 2 pour l'immobiliser à l'intérieur du logement 16 et la déformer radialement du fait de sa fente 21 pour venir serrer l'axe 32 à l'intérieur de l'alésage 20. On constate que les points d'impact des vis 19 avec la sphère 2 se situent au-dessus de ses axes principaux de manière à l'empêcher de remonter sous des efforts de traction.

On a illustré en fig. 4, 5, 6 et 7 des variantes d'exécution de la prothèse modulaire illustrée en fig. 1 à 3. Sur ces figures, les éléments correspondant à ceux de fig. 1 à 3 ont été référencés par les mêmes chiffres et les mêmes indices.

Le but de la variante illustrée en fig. 4 et 5 consiste à prévoir une sphère 2' comportant un état de surface permettant une indexation de chacune de ses positions par rapport à la queue humérale 1.

Le logement cylindrique 16 prévu à l'intérieur de la partie métaphysaire 11 de la queue 1 comporte sur sa portée sphérique 16a des reliefs 16b régulièrement répartis sur le pourtour de la circonférence du logement 16.

La sphère 2' est percée d'un alésage central 20' dans lequel débouche une fente 21' tandis que son état de surface est recouvert d'une série de cavités 24' en portion de sphère de diamètre sensiblement identique à ceux des reliefs 16b prévus sur la portée sphérique 16a du logement 16. Egalement, la sphère 2' comporte à chaque pôle des plats 22' et 23' réduisant son encombrement à l'intérieur du logement 16.

On constate que les cavités 24' coopèrent avec les reliefs 16b du logement 16 permettant une indexation angulaire de la sphère 2' avant son immobilisation avec l'axe 32 de la calotte 3 par l'intermédiaire des vis de serrage 19.

La seconde variante illustrée en fig. 6 et 7 est prévue pour pouvoir utiliser les calottes sphériques décrites dans la demande de brevet N° 2 685 633 appartenant à la Demanderesse.

En effet, le logement cylindrique 16 à portée spérique 16a est prévu pour recevoir une sphère 4 solidaire d'une collerette circulaire 40 au centre de laquelle s'élève un pion tronconique 41 à côté duquel est ménagé un trou borgne 42 destiné à recevoir un téton 5.

A proximité du pion tronconique 41 est prévu un autre trou débouchant 43 étagé qui permet la mise en place d'une vis 6 dont on verra mieux plus loin l'utilisation.

Une calotte hémisphérique 3' comprend une face extérieure 30' parfaitement polie limitée par une base 31'. Dans cette dernière, on a ménagé de manière excentrée un emboîtement circulaire 32' dont le diamètre correspond au jeu près à celui de la colerette 40. Au centre de cet emboîtement est ménagé un alésage tronconique 33' de diamètre correspondant à celui du pion 41 de la collerette 40. Une série de huit trous taraudés 34' est réalisée dans le fond de l'emboitement 32' de manière concentrique au centre de celui-ci.

Le montage de la calotte hémisphérique 3' par rapport à la sphère 4 s'effectue par l'engagement du pion 41 dans l'alésage 33', la collerette 40 venant s'ajuster dans l'emboîtement 32', tandis que le téton 5 solidaire du trou 42 vient s'enfiler dans l'un des trous 34' . L'immobilisation de la calotte 3' sur la sphère 4 est réalisée au moyen de la vis 6 qui vient coopérer avec le trou taraudé 34' correspondant, après avoir traversé le trou étagé 43.

La calotte hémisphérique 3' étant montée sur la sphère 4, il suffit de fixer l'ensemble sur la queue 1. Pour cela, la sphère 4 est introduite dans le logement cylindrique 16 de manière à venir en appui contre la portée sphérique 16a, tandis que les vis de pression 19 sont serrées pour venir en contact de ladite sphère afin de l'immobiliser dans la position angulaire recherchée.

On remarque que la surface extérieure de la sphère 4 peut être recouverte d'une série de cavités identiques à celles 24' de la sphère 2' pour coopérer avec les reliefs 16b du logement 16 afin de permettre une indexation de ladite sphère 4 avant sa fixation.

On a représenté en fig. 8 à 13 une troisième variante de la prothèse modulaire suivant l'invention permettant d'utiliser les calottes hémisphériques décrites dans la demande de brevet N° 2 685 633 appartenant à la demanderesse.

Sur les figures 8 à 13, les éléments correspondant à ceux de fig. 6 et 7 ont été référencés par les mêmes chiffres et les mêmes indices. La prothèse humérale comprend une queue 1', une sphère 4 et une calotte hémisphérique 3' .

La queue 1' comprend une tige 10' de section circulaire qui s'engage dans le canal huméral et une partie métaphysaire 11' présentant un profil évasé. Dans le plan sagittal, la partie métaphysaire 11' comporte des flans 12' et 13' légèrement inclinés. En outre, la partie métaphysaire 11' est pourvue d'un ou plusieurs ailerons 14' disposés sur la face externe et/ou latérale de la tige 10' et qui comprennent au moins une perforation 14'a permettant, dans le cas de fracture, une reconstitution de l'extrémité supérieure de l'humérus autour de la prothèse.

La tige 10', et plus particulièrement la partie métaphysaire 11', se termine par une face inclinée 15' par rapport à l'axe longitudinal de ladite tige. La face inclinée 15' est percée d'un logement 16' présentant une portée sphérique 16'a destinée à servir d'appui à la sphère 4, comme on le verra mieux plus loin.

Dans la partie métaphysaire 11' et sur le flanc 12' est ménagée une ouverture 16'b en forme de C débouchant à l'intérieur du logement 16' . A l'opposé de l'ouverture 16'b et sur le flanc 13' est percé un trou 16'c débouchant à l'intérieur du logement 16' . Ce dernier comporte perpendiculairement à l'ouverture 16'b deux faces parallèles 16'd, 16'e qui sont disposées également perpendiculairement à la face inclinée 15' . La distance séparant les faces 16'd et 16'e est inférieure au diamètre de la portée sphérique 16'a . On constate que cette dernière se prolonge au-dessus de ses axes principaux pour définir une portée faisant plus d'une demi-sphère.

La partie métaphysaire 11' est percée d'au moins un trou taraudé 17' qui débouche à l'intérieur du logement 16'et plus particulièrement à l'intérieur de la portée sphérique 16'a. Le trou 17' est prévu pour recevoir une vis de pression 19' sans tête pour l'immobilisation de la sphère 4. Les axes principaux du trou 17' sont prévus soit obliques et inclinés par rapport aux axes du logement 16', soit parallèles à ceux définissant la face inclinée 15' .

Le logement 16' à portée sphérique 16'a est prévu pour recevoir une sphère 4 solidaire d'une collerette circulaire 40 au centre de laquelle s'élève un pion tronconique 41 à côté duquel est ménagé un trou borgne 42 destiné à recevoir un téton 5 non représenté.

A proximité du pion tronconique 41 est prévu un autre trou débouchant 43 étagé qui permet la mise en place d'une vis 6 non représentée.

En outre la sphère 4 présente sur sa périphérie des méplats 44, 45 disposés à l'opposé l'un de l'autre. On observe que la distance séparant les deux méplats opposés 44 et 45 est légèrement inférieure à celle prévue entre les faces parallèles 16'd et 16'e.

Le pôle de la sphère 4 opposé à la collerette 40 est usiné pour former un plat 47 qui est percé en son milieu d'un trou taraudé 47a.

Le trou taraudé 47a communique coaxialement avec un trou lisse 48 qui est ménagé dans le pion tronconique 41 et la collerette 40. Le trou lisse 48 débouche à l'extérieur dans la partie supérieure du pion tronconique 41 pour permettre l'engagement d'une clé 49.

Le trou taraudé 47a est prévu pour recevoir une vis de pression 47b sans tête qui est actionnée par la clé 49 comme on le verra mieux plus loin.

La mise en place et l'immobilisation de la calotte 3' sur la sphère 4 est réalisée au moyen de la vis 6 comme cela est déjà décrit précédemment en figures 6 et 7.

La calotte hémisphérique 3' étant montée sur la sphère 4, il suffit de fixer l'ensemble sur la queue 1'. Pour cela, la sphère 4 est introduite dans le logement cylindrique 16' de manière à venir en appui contre la portée sphérique 16'a.

La mise en place et la fixation de la sphère 4 sur la queue 1' est obtenue de la manière suivante : on présente méplats 44 et 45 parallèlement aux faces 16'd et 16'e pour permettre l'introduction de la sphère 4 dans le logement 16' (fig. 10).

On remarque que dans cette position la collerette 40 est disposée parallèlement à l'ouverture 16'b, tandis que le plat 47 se trouve en face du trou 16'c.

On bascule ensuite la sphère 4 à l'intérieur du logement 16', pour que la collerette 40 soit placée au-dessus de la face inclinée 15' de la queue 1' (fig. 12).

On pivote la sphère 4 autour de son axe, pour que les méplats 44, 45 se trouvent respectivement en face de l'ouverture 16'b et du trou 16'c (fig. 11, 13).

On serre les vis de pression 19' et 47b pour immobiliser la sphère 4 dans la position angulaire recherchée (fig. 11, 13).

On remarque que la vis de pression 47b est actionnée par la clé 49 qui traverse le pion 41 et la collerette 40, afin que ladite vis vienne en appui contre le fond de la portée sphérique 16'a. Le serrage de la vis 47b permet de venir plaquer et bloquer la sphère 4 sur la partie supérieure de la portée sphérique 16'a.

La vis 19' vient en contact de la surface sphérique pour permettre le blocage en rotation de la sphère 4 autour de son axe.

On constate que la prothèse modulaire suivant l'invention permet au chirurgien de régler la calotte hémisphérique 3, 3' dans plusieurs plans différents par rapport à l'axe principal de la queue 1, 1' de manière à répondre parfaitement à toutes les anatomies. Ce genre de prothèse modulaire évite de tenir en stock un certain nombre d'entretoises pour régler l'inclinaison par rapport à l'axe de la queue.

Il doit d'ailleurs être entendu que la description qui précède n'a été donnée qu'à titre d'exemple et qu'elle ne limite nullement le domaine de l'invention dont on ne sortirait pas en remplaçant les détails d'exécution décrits par tous autres équivalents.

## Revendications

1. Prothèse modulaire comprenant une queue (10, 10') pouvant s'ancrer dans le canal huméral et une calotte (3, 3') à profil sensiblement hémisphérique propre à coopérer avec la glène de l'épaule, caractérisée en ce que la queue humérale (1 ; 1') est pourvue dans sa partie métaphysaire (11 ;, 11') d'un logement (16 ; 16') à fond sphérique (16a, 16'a) dans lequel est introduite une sphère (2, 2' ; 4) prévue pour recevoir la calotte hémisphérique (3, 3'), tandis que des moyens de serrage (19, 19', 47b, 6) permettent leur immobilisation dans une position angulaire déterminée par rapport à l'axe de la queue humérale (1 ; 1').

2. Prothèse modulaire suivant la revendication 1, caractérisée en ce que la sphère (2) comporte en son milieu un alésage (20) dans lequel débouche au moins une fente radiale (21) tandis qu'elle comprend à chaque pôle un plat (22, 23) limitant son encombrement à l'intérieur du logement cylindrique (16).

3. Prothèse modulaire suivant la revendication 1, caractérisée en ce que le logement cylindrique (16) et plus particulièrement son fond sphérique (16a) présente au moins un relief (16b) en forme de portion de sphère pour l'indexation angulaire d'une sphère (2') dont la surface extérieure est recouverte d'une série de cavités (24') de forme complémentaire à celle dudit relief.

4. Prothèse modulaire suivant la revendication 1, caractérisée en ce que la queue (1) comporte dans sa partie métaphysaire (11) des trous taraudés (17, 18) prévus pour recevoir chacun une vis de pression (19) permettant l'immobilisation de la sphère (2, 2', 4) à l'intérieur du logement cylindrique (16).

5. Prothèse modulaire suivant la revendication 1, caractérisée en ce que la calotte hémisphérique (3) comprend une face extérieure (30) limitée par une base (31) qui est solidaire de manière excentrée d'un axe vertical (32).

6. Prothèse modulaire suivant la revendication 1, caractérisée en ce que l'axe (32) de la calotte hémiphérique (3) est prévu pour coopérer avec l'alésage (20, 20') des sphères (2, 2') pour permettre son réglage angulaire par rapport à l'axe de la queue (1).

7. Prothèse modulaire suivant la revendication 1, caractérisée en ce que l'axe (32) de la calotte hémisphérique (3) est immobilisé à l'intérieur de l'alésage (20, 20') de la sphère (2, 2') par l'intermédiaire des vis de serrage (19) et de la fente radiale (21, 21').

8. Prothèse modulaire suivant la revendication 7, caractérisé en ce que l'axe (32) permet le réglage en hauteur de la calotte hémisphérique (3).

9. Prothèse modulaire suivant la revendication 1, caractérisée en ce que la calotte hémisphérique (3') comprend une face extérieure (30') délimitée par une base (31') sur laquelle est ménagée de manière excentrée un emboîtement circulaire (32') au milieu duquel est prévu un alésage tronconique (33'), tandis qu'une série de huit trous taraudés (34') sont ménagés concentriquement autour de l'alésage tronconique (33').

10. Prothèse modulaire suivant la revendication 9, caractérisée en ce que la sphère (4) est solidaire d'une collerette circulaire (40) qui s'engage à jeu réduit dans l'emboîtement excentré (32') de la base (3l') de la calotte hémisphérique (3'), tandis qu'un pion tronconique (41) vient se coincer dans l'alésage (33') de même profil situé au centre de l'emboîtement (32') et une vis de serrage (6) coopère avec l'un des trous taraudés (34')

11. Prothèse modulaire suivant la revendication 1, caractérisée en ce que le logement (16') comporte une ouverture (16'b) en forme de C, un trou (16'c) percé à l'opposé de ladite ouverture, deux faces parallèles (16'd et 16'e) disposées perpendiculairement à ladite ouverture et une portée sphérique (16'a).

12. Prothèse modulaire suivant la revendication 11, caractérisée en ce que la distance séparant les faces parallèles (16'd et 16'e) est inférieure au diamètre de la portée sphérique (16'a).

13. Prothèse modulaire suivant la revendication 11, caractérisée en ce que la portée sphérique (16'a) se prolonge au-dessus de ses axes principaux.

14. Prothèse modulaire suivant la revendication 1, caractérisée en ce que la sphère (4) comporte des méplats (44, 45 et 46) dont deux au moins sont disposés parallèlement l'un à l'autre.

15. Prothèse modulaire suivant la revendication 14, caractérisée en ce que la distance séparant les méplats (44 et 45) est inférieure à celle prévue entre les faces (16'd, 16'e) du logement (16').

16. Prothèse modulaire suivant la revendication 1, caractérisée en ce que la sphère (4) comporte à son pôle un trou taraudé (47a) qui communique coaxialement avec un trou lisse et débouchant (48) qui est percé dans le pion tronconique (41) de la collerette (40).

17. Prothèse modulaire suivant la revendication 1, caractérisée en ce que les vis de pression (19', 47b) permettent l'immobilisation angulaire de la sphère (4) à l'intérieur du logement (16').

## Claims

1. Modular prosthesis comprising a stem (10, 10') which is adapted to anchor itself in the humeral canal and a calotte-type helmet (3, 3') having a substantially hemispherical profile which is adapted to cooperate with the shoulder socket, characterised in that the stem of the humerus (1; 1') is provided in its metaphysial part (11; 11') with a seat (16; 16') having a spherical base (16a; 16 'a) into which is introduced a sphere (2, 2'; 4) provided to receive the hemispherical helmet (3, 3'), while locking means (19, 19', 47b, 6) enable these to be immobilised in an angular position determined with respect to the axis of the stem of the humerus (1; 1').

2. Modular prosthesis according to claim 1, characterised in that the sphere (2) has in its middle a bore (20) into which at least one radial slot (21) opens out, while at each pole said sphere features a flat face (22, 23) which limits how much space it takes up inside the cylindrical seat (16).

3. Modular prosthesis according to claim 1, characterised in that the cylindrical seat (16) and more particularly the spherical base (16a) thereof presents at least one prominence (16b) in the form of a spherical portion for the angular indexing of a sphere (2') whose external surface is covered with a series of cavities (24') of a shape complementing that of said prominence.

4. Modular prosthesis according to claim 1, characterised in that in its metaphysial part (11) the stem (1) has tapped holes (17, 18) provided to respectively receive a press-screw (19) enabling the sphere (2, 2', 4) to be immobilised inside the cylindrical seat (16).

5. Modular prosthesis according to claim 1, characterised in that the hemispherical helmet (3) has an outer face (30) limited by a base (31) which is eccentrically integral with a vertical axis (32).

6. Modular prosthesis according to claim 1, characterised in that the axis (32) of the hemispherical helmet (3) is provided to cooperate with the bore (20, 20') of the spheres (2, 2') so as to allow the angle thereof to be adjusted with respect to the axis of the stem (1).

7. Modular prosthesis according to claim 1, characterised in that the axis (32) of the hemispherical helmet (3) is immobilised inside the bore (20, 20') of the sphere (2, 2') by means of locking screws (19) and the radial slot (21, 21').

8. Modular prosthesis according to claim 7, characterised in that the axis (32) allows the height of the hemispherical helmet (3) to be adjusted.

9. Modular prosthesis according to claim 1, characterised in that the hemispherical helmet (3') features an outer face (30') delimited by a base (31') on which is eccentrically disposed a circular nesting arrangement (32') in the middle of which a tapered bore (33') is provided, while a series of eight tapped holes (34') are disposed concentrically about the tapered bore (33').

10. Modular prosthesis according to claim 9, characterised in that the sphere (4) is rigidly joined to a circular flange (40) which engages with minor clearance in the eccentric nesting arrangement (32') of the base (31') of the hemispherical helmet (3'), while a tapered spigot (41) wedges itself in the bore (33') of the same profile which is situated at the centre of the nesting arrangement (32') and a locking screw (6) cooperates with one of the tapped holes (34').

11. Modular prosthesis according to claim 1, characterised in that the nesting arrangement (16') features a C-shaped opening (16'b), a drilled hole (16'c) opposite said opening, two parallel faces (16'd and 16'e) disposed perpendicularly to said opening and a spherical bearing surface (16'a).

12. Modular prosthesis according to claim 11, characterised in that the distance separating the parallel faces (16'd and 16'e) is less than the diameter of the spherical bearing surface (16'a).

13. Modular prosthesis according to claim 11, characterised in that the spherical bearing surface (16'a) extends above its main axes.

14. Modular prosthesis according to claim 1, characterised in that the sphere (4) has flat surfaces (44, 45 and 46), at least two of which are disposed parallel to one another.

15. Modular prosthesis according to claim 14, characterised in that the distance separating the flat surfaces (44 and 45) is less than the distance provided between the faces (16'd, 16'e) of the seat (16').

16. Modular prosthesis according to claim 1, characterised in that the sphere (4) has at its pole a tapped hole (47a) which communicates coaxially with a smooth-bored exit hole (48) which is drilled in the tapered spigot (41) of the flange (40).

17. Modular prosthesis according to claim 1, characterised in that the press-screws (19', 47b) allow angular immobilisation of the sphere (4) inside the seat (16').

## Patentansprüche

1. Modulare Prothese umfassend einen Stiel (10,10'), der im Humeruskanal verankerbar ist, und eine Kappe (3,3') mit einem im wesentlichen halbkugelförmigen Profil, die mit der Gelenkpfanne der Schulter Zusammenwirken kann,
dadurch **gekennzeichnet,**
daß der humerale Schaft (1,1') an seinem metaphysären Abschnitt (11,11') einen Lagersitz (16,16') mit einem kugelförmigen Boden (16a,16a') aufweist, in den eine Kugel (2,2';4) einsetzbar ist, die der Aufnahme der halbkugelförmigen Kappe (3,3') dient, wobei Verriegelungsmittel (19,19',47b,6) deren Fixierung in einer zur Achse des humeralen Schafts (1,1') bestimmten Winkelstellung ermöglichen.

2. Modulare Prothese nach Anspruch 1,
dadurch **gekennzeichnet,**
daß die Kugel (2) in ihrer Mitte eine Bohrung (20) aufweist, in die mindestens ein sich radial erstreckender Schlitz (21) mündet, wobei die Kugel (2) an jedem Pol eine ebene Fläche (22,23) aufweist, die deren Blockierung im Inneren des zylindrischen Lagersitzes (16) begrenzt.

3. Modulare Prothese nach Anspruch 1,
dadurch **gekennzeichnet,**
daß der zylindrische Lagersitz (16) und insbesondere sein kugelförmiger Boden (16a) mindestens einen kugelabschnittsförmigen Vorsprung (16b) zur Festlegung des Neigungswinkels einer Kugel (2') aufweisen, deren Außenfläche mit mehreren Kavitäten (24') überzogen ist, die eine zur Form des Vorsprungs komplementäre Form aufweisen.

4. Modulare Prothese nach Anspruch 1,
dadurch **gekennzeichnet,**
daß der Schaft (1) an seinem metaphysären Abschnitt (11) Gewindebohrungen (17,18) aufweist, die jeweils der Aufnahme einer Feststellschraube (19) dienen, die die Fixierung der Kugel (2,2';4) im Inneren des zylindrischen Lagersitzes (16) ermöglichen.

5. Modulare Prothese nach Anspruch 1,
dadurch **gekennzeichnet,**
daß die halbkugelförmige Kappe (3) eine Außenfläche (30) aufweist, die von einer Grundfläche (31) begrenzt ist, die mit einer vertikalen Achse (32) fest und exzentrisch zu dieser verbunden ist.

6. Modulare Prothese nach Anspruch 1,
dadurch **gekennzeichnet,**
daß die Achse (32) der halbkugelförmigen Kappe (3) mit der Bohrung (20,20') der Kugeln (2,2') so zusammenwirkt, daß deren Neigung zur Achse des Schafts (1) einstellbar ist.

7. Modulare Prothese nach Anspruch 1,
dadurch **gekennzeichnet,**
daß die Achse (32) der halbkugelförmigen Kappe (3) im Inneren der Bohrung (20,20') der Kugel (2,2') mittels der Stellschrauben (19) und des radialen Schlitzes (21,21') verriegelbar ist.

8. Modulare Prothese nach Anspruch 7,
dadurch **gekennzeichnet,**
daß die Achse (32) eine Höheneinstellung der halbkugelförmigen Kappe (3) ermöglicht.

9. Modulare Prothese nach Anspruch 1,
dadurch **gekennzeichnet,**
daß die halbkugelförmige Kappe (3') eine Außenfläche (30') aufweist, die von einer Grundfläche (31') begrenzt ist, in der eine kreisförmige Vertiefung (32') exzentrisch angeordnet ist, die in ihrer Mitte eine kegelstumpfartige Bohrung (33') aufweist, um die acht Gewindebohrungen (34') konzentrisch angeordnet sind.

10. Modulare Prothese nach Anspruch 9,
dadurch **gekennzeichnet,**
daß die Kugel (4) mit einem kreisförmigen Kragen (40) fest verbunden ist, der in die kreisförmige Vertiefung (32') in der Grundfläche (31') der halbkugelförmigen Kappe (3') mit reduziertem Spiel einsetzbar ist, wobei ein kegelstumpfartiger Zapfen (41) in einer Bohrung (33') gleichen Profils in der Mitte der Vertiefung (32') zum Eingriff kommt und eine Klemmschraube (6) mit einer der Gewindebohrungen (34') zusammenwirkt.

11. Modulare Prothese nach Anspruch 1,
dadurch **gekennzeichnet,**
daß der Lagersitz (16') eine C-förmige Öffnung (16'b), dieser gegenüberliegend ein Loch (16'c), senkrecht zu dieser zwei parallele Seitenflächen (16'd und 16'e) und einen kugelförmigen Bereich (16'a) aufweist.

12. Modulare Prothese nach Anspruch 11,
dadurch **gekennzeichnet,**
daß der Abstand zwischen den parallelen Seitenflächen (16'd und 16'e) kleiner ist als der Durchmesser des kugelförmigen Bereichs (16'a).

13. Modulare Prothese nach Anspruch 11,
dadurch **gekennzeichnet,**
daß sich der kugelförmige Bereich (16'a) oberhalb seiner Hauptachsen ausdehnt.

14. Modulare Prothese nach Anspruch 1,
dadurch **gekennzeichnet,**
daß die Kugel (4) Abflachungen (44,45,46) aufweist, von denen mindestens zwei parallel zueinander angeordnet sind.

15. Modulare Prothese nach Anspruch 14,
dadurch **gekennzeichnet,**
daß der Abstand zwischen den Abflachungen (44,45) kleiner ist als derjenige zwischen den Seitenflächen (16'd,16'e) des Lagersitzes (16').

16. Modulare Prothese nach Anspruch 1,
dadurch **gekennzeichnet,**
daß die Kugel (4) an ihrem Pol ein Gewindeloch (47a) aufweist, das mit einem glatten Durchgangsloch (48) koaxial in Verbindung steht, das in dem kegelstumpfartigen Zapfen (41) des Kragens (40) verläuft.

17. Modulare Prothese nach Anspruch 1,
dadurch **gekennzeichnet,**
daß die Feststellschrauben (19',47b) die Winkelfixierung der Kugel (4) im Inneren des Lagersitzes (16') ermöglichen.
